# EUROPÄISCHE PATENTSCHRIFT

(11) **EP 4 192 367 B1**
(45) Veröffentlichungstag und Bekanntmachung des Hinweises auf die Patenterteilung: **12.06.2024**
(21) Anmeldenummer: 22748330.2
(22) Anmeldetag: 11.07.2022
(51) Int. Cl.: A61B 17/16

(54) **AUTOMATISCHE RFID-WERKZEUGKUPPLUNG**
AUTOMATIC RFID TOOL COUPLING
COUPLAGE D'OUTIL RFID AUTOMATIQUE

(30) Priorität: 16.07.2021 DE 102021118412
(43) Veröffentlichungstag der Anmeldung: 14.06.2023
(73) Patentinhaber: Aesculap AG, 78532 Tuttlingen (DE)
(72) Erfinder: LENZENHUBER, Frederick, 78532 Tuttlingen (DE); BÜRK, André, 78056 Villingen-Schwenningen (DE)
(74) Vertreter: Winter, Brandl - Partnerschaft mbB
(86) Internationale Anmeldenummer: PCT/EP2022/069312
(87) Internationale Veröffentlichungsnummer: WO 2023/285375

(56) Entgegenhaltungen:
- WO-A2-2020/079268
- DE-B3- 10 311 455
- US-A- 5 584 689
- US-A1- 2003 023 256
- US-A1- 2018 256 287

## Beschreibung

### Technisches Gebiet

Die vorliegende Offenbarung betrifft ein medizinisches/chirurgisches Werkzeug oder Instrument, insbesondere ein über ein Identifizierungselement identifizierbares Werkzeug, das mit einer Schaftgeometrie versehen ist, die sowohl eine Axialsicherung als auch eine Drehmomentübertragung auf das Werkzeug ermöglicht.

In der Medizin werden heute bereits Werkzeuge eingesetzt, deren Arbeitsende automatisch erkannt werden kann. Das Arbeitsende stellt den funktionalen Teil des Werkzeugs dar und kann anwendungsspezifisch in vielfältiger Weise ausgeformt sein. Werkzeuge der hierin in Bezug genommenen Art beinhalten allgemein auf dem Gebiet der Medizin verwendbare Werkzeuge, Instrumente, Sprühdüsen, HF-Spitzen, Ultraschallklingen und dergleichen.

Eine solche Erkennung des Arbeitsendes geschieht mittels an dem Werkzeug angebrachter, digital auslesbarer Datenspeicher, welche als Identifizierungselement fungieren und eine Identifizierung des Werkzeugs bzw. des Arbeitsendes des Werkzeugs ermöglichen.

Die Unterbringung der digital auslesbaren Datenspeicher an/in dem Werkzeug stellt die Industrie jedoch vor Probleme. Medizinische Werkzeuge sind in der Regel kompakt und müssen trotz einer möglichst filigranen Bauweise harten Einsatzbedingungen standhalten. Das Anbringen eines digital auslesbaren Datenspeichers in/an einem Werkzeug sorgt jedoch dafür, dass die Struktur des Werkzeugs geschwächt wird und Kerbspannungen in dem Werkzeug entstehen können. Um dem entgegen zu wirken, werden die Werkzeuge und insbesondere die drehmomentübertragenden Abschnitte des Werkzeugs robuster ausgeführt, wodurch sich die Abmessungen des Werkzeugs vergrößern und damit die Werkzeuge unhandlicher in der Handhabung werden.

Die US 5 584 689 A offenbart medizinische Werkzeuge mit muldenförmigen Einbuchtungen zur Kraftübertragung.

Die US 2003 / 023 256 A1 offenbart medizinische Werkzeuge mit geometrischen Kraftübertragungsabschnitten in unterschiedlichen Ausführungen.

Die WO 2020 / 079 268 A2 offenbart ein medizinisches Werkzeug mit einer Hülse zur Aufnahme eines Erkennungselements.

Die DE 103 11 455 B3 offenbart eine Kupplung für ein chirurgisches Drehantriebs-Handstück mit einem in dem Handstück gelagerten und von diesem drehend antreibbarren Werkzeug.

DIE US 2018 / 256 287 A1 offenbart ein System zur Erkennung medizinischer Werkzeuge.

Es besteht daher der Bedarf an einer Möglichkeit, einen digital auslesbaren Datenspeicher/ein Identifikationselement an/in einem Werkzeug unterzubringen, wobei, trotz kompakter/filigraner Bauweise des Werkzeugs, ein großes Moment von einer Krafteinleitungsstelle/der Schaftgeometrie auf ein Arbeitsende übertragen werden kann.

Diese Aufgabe wird durch ein in Anspruch 1 beschriebenes, Werkzeug sowie das in Anspruch 9 beschriebene Instrumentenset gelöst. Vorteilhafte Ausführungsformen sind in den Unteransprüchen beschrieben.

Die vorliegende Offenbarung beschreibt ein medizinisches Werkzeug mit einem Werkzeugschaft, der in einem Schaftbereich eine Schaftgeometrie zur Drehmomenteinleitung und Axialsicherung aufweist. Diese Schaftgeometrie zur Drehmomenteinleitung und gleichzeitigen Axialsicherung besteht einzig aus zumindest einer (oder mehreren) mulden- oder badewannenförmigen Vertiefung(en)/Einbuchtung(en)/Rastausbuchtung(en) in der äußeren Mantelfläche des Werkzeugschafts. Das Werkzeug beinhaltet einen proximal zu der Schaftgeometrie angeordneten Ausrichtungsabschnitt. Weiterhin weist das Werkzeug ein Identifizierungselement auf, welches in einer Hülse angeordnet ist, welche den Ausrichtungsabschnitt beinhaltet.

In anderen Worten weist das Werkzeug der vorliegenden Offenbarung den Schaftbereich auf dem bzw. längs des Werkzeugschafts auf, wobei der Schaftbereich mit einer Schaftgeometrie versehen ist, die es ermöglicht, sowohl das Drehmoment auf das Werkzeug zu übertragen als auch das Werkzeug axial zu sichern. Dies wird erfindungsgemäß einzig/ausschließlich über die mindestens eine muldenförmige Einbuchtung in der Oberfläche des Werkzeugschafts in dem Schaftbereich erreicht.

Kern der Offenbarung ist demnach, dass sowohl die Drehmomentübertragung als auch die axiale Sicherung des Werkzeugs einzig und allein über mindestens eine muldenförmige Vertiefung in der Mantelfläche des Werkzeugschafts gewährleistet wird. Eine Aufteilung dieser beiden Funktionen in zwei in Schaftlängsrichtung beabstandete Schaftbereiche/Funktionsabschnitte erfolgt demnach nicht.

Durch das Zusammenlegen der Axialsicherung und der Drehmomentübertragung auf nur eine (einzige) Geometrie ist es möglich, das Werkzeug kompakter zu gestalten und trotzdem eine hohe Drehmomentübertragung von der Drehmomenteinleitungsstelle in Form der Schaftgeometrie bis zum Arbeitsende zu gewährleisten. Zusätzlich wird durch die muldenförmige Geometrie der Schaftgeometrie eine Vermeidung von Kerbspannungen an dem Werkzeugschaft erzielt und die Torsionssteifigkeit des Werkzeugschafts ist erhöht.

In einem ersten bevorzugten Aspekt besteht die Schaftgeometrie des Werkzeugs aus mehreren, gleichmäßig über dem Umfang des Werkzeugs verteilten, muldenförmigen Vertiefungen/Rastausbuchtungen.

In anderen Worten weist die Schaftgeometrie mehr als eine muldenförmige Vertiefung entlang eines radialen Abschnitts des Werkzeugschafts auf, wobei jede muldenförmige Vertiefung radial den gleichen Winkelabstand zu ihrer benachbarten muldenförmigen Vertiefung aufweist.

In nochmals anderen Worten ist der Abstand in Umfangsrichtung von jeder muldenförmigen Vertiefung zu ihrer benachbarten muldenförmigen Vertiefung konstant über den gesamten Umfang des Werkzeugs.

Durch mehrere, gleichmäßig über den Umfang des Werkzeugs verteilte, muldenförmige Vertiefungen ist eine gleichmäßige Einleitung eines Antriebsdrehmoments in das Werkzeug gewährleistet. Hierdurch sind die Belastung des Werkzeugs sowie die Belastung einer Lagerung des Werkzeugs reduziert.

In einem weiteren Aspekt weist das Werkzeug einen proximal zu der Schaftgeometrie angeordneten Ausrichtungsabschnitt auf, wobei der Ausrichtungsabschnitt eine pyramidenförmige Geometrie aufweist. Insbesondere befindet sich die Pyramidenspitze des Ausrichtungsabschnitts auf der Mittelfaser des Werkzeugs.

In anderen Worten ist auf der von dem Arbeitsende des Werkzeugs gegenüberliegenden Seite der Schaftgeometrie der Ausrichtungsabschnitt vorgesehen, der pyramidenförmig ausgebildet ist. Die auf der Mittelfaser des Werkzeugs liegende Pyramidenspitze bildet den am weitesten von dem Arbeitsende entferntesten Punkt des Werkzeugs.

In einem weiteren bevorzugten Aspekt entspricht eine Anzahl von Pyramidenseiten einer Anzahl von muldenförmigen Vertiefungen.

In anderen Worten ist die Anzahl der Pyramidenseiten des Ausrichtungsabschnitts des Werkzeugs auf die Anzahl der muldenförmigen Vertiefungen der Schaftgeometrie des Werkzeugs abgestimmt. Vorzugsweise beträgt die Anzahl der Pyramidenseiten sowie die Anzahl der muldenförmigen Vertiefungen drei.

In einem weiteren bevorzugten Aspekt ist die Anzahl der muldenförmigen Einbuchtungen ein ganzzahliges Vielfaches der Anzahl der Pyramidenseiten.

In einem weiteren Aspekt weist das Werkzeug ein Identifizierungselement auf, welches in einer ((stoff-)einstückigen) Hülse angeordnet ist, die den Ausrichtungsabschnitt beinhaltet.

In anderen Worten ist an einem proximalen Abschnitt des Werkzeugs die vorzugsweise (stoff-)einstückige Hülse an dem Werkzeug angebracht, die vorgesehen und ausgebildet ist, das Identifizierungselement aufzunehmen. An der proximalen Stirnseite der Hülse befindet sich der Ausrichtungsabschnitt. Die Hülse ist vorzugsweise kraft- und/oder formschlüssig mit dem Werkzeugschaft verbunden.

Das Identifizierungselement ermöglicht eine Erkennung des individuellen Werkzeugs. Darüber lassen sich reale Nutzungsdauern einzelner Werkzeuge in Kombination mit einem Handstück erfassen und dokumentieren. In einem weiteren Schritt ist es so möglich, die Lebensdauer der Werkzeuge sicher zu ermitteln. Zusätzlich können werkzeugbezogene (Einsatz-)Parameter auf dem Identifizierungselement hinterlegt werden, welche dann vorzugsweise automatisch an eine Bedien- und Steuereinheit weitergegeben werden, welche dann die für das Werkzeug benötigten Einstellungen automatisch vornimmt. Auch sind neue Geschäftsmodelle durch die werkzeugspezifische Identifikation wie beispielsweise pay per use und dergleichen denkbar.

Weiterhin ist vorstellbar, nach jedem Einsatz eines Werkzeugs alle während der Einsatzdauer auftretenden Situationen, wie beispielsweise eine Überschreitung der zulässigen Drehzahl für das Werkzeug, auf dem Identifizierungselement zu speichern und so im Falle eines Ausfalls des Werkzeugs eine Ursachennachforschung zu erleichtern. Bevorzugt ist das Identifizierungselement ein RFID-Element, das beispielsweise über NFC ansprechbar ist.

In einem weiteren bevorzugten Aspekt besteht die Hülse vorzugsweise aus Kunststoff, was eine möglichst gute Ansprechbarkeit des Identifizierungselements gewährleistet. Alternativ besteht die Hülse aus Metall, was den Schutz des Identifizierungselements zusätzlich erhöht. Um trotzdem eine gute Ansprechbarkeit des Identifizierungselements zu erzielen, sind in der Hülse Schlitze vorgesehen, welche optional mit einer Vergussmasse oder Kunststoffeinlegeteilen oder dergleichen verschlossen sind.

In einem weiteren bevorzugten Aspekt weist die Hülse einen Innenraum auf, der vorgesehen und ausgebildet ist, ein Identifizierungselement von vorzugsweise 1,4 mm x 8 mm aufzunehmen.

In anderen Worten ist die Hülse so ausgebildet, dass das Identifizierungselement mit einem Durchmesser zwischen 0,8 mm und 5 mm, vorzugsweise einem Durchmesser zwischen 1,2 mm und 1,6 mm und insbesondere einem Durchmesser von 1,4 mm in dem von der Hülse in einem montierten Zustand eingeschlossenen Innenraum positioniert ist. Die Länge des Identifizierungselements beträgt 4 mm bis 15 mm, vorzugsweise 6 mm bis 10 mm und insbesondere 8 mm. Vorzugsweise ist das Identifizierungselement spielfrei in dem Innenraum der Hülse positioniert.

Das Identifizierungselement ist vorzugsweise vollständig in der Hülse untergebracht und damit gegen Umwelteinflüsse geschützt. Gleichzeitig ist gewährleistet, dass das Identifizierungselement keinen Antriebskräften ausgesetzt ist, welche das Identifizierungselement beschädigen könnten. Ein solch modularer Aufbau ermöglicht es, das Identifizierungselement kostengünstig an/in dem Werkzeug anzubringen.

In einem weiteren bevorzugten Aspekt ist der Ausrichtungsabschnitt in Relation zu der Schaftgeometrie vorbestimmt orientiert.

In anderen Worten ist die radiale Ausrichtung des Ausrichtungsabschnitts auf die radiale Ausrichtung der Schaftgeometrie abgestimmt.

In nochmals anderen Worten ist ein Mittelpunkt der Pyramidenfläche des Ausrichtungsabschnitts radial gleich orientiert wie der Mittelpunkt der entsprechenden muldenförmigen Vertiefung.

In einem weiteren bevorzugten Aspekt wird die vorbestimmte Orientierung über eine Positionierhilfe zwischen Ausrichtungsabschnitt und Schaftgeometrie festgelegt.

In anderen Worten ist die Orientierung zwischen dem Ausrichtungsabschnitt und der Schaftgeometrie durch eine Positionierhilfe, vorzugsweise nach einem Schlüssel-Schloss-Prinzip oder einem Verstiftungsprinzip, definiert. Die Positionierhilfe verhindert eine falsche Montage des Ausrichtungsabschnitts in Relation zu der Schaftgeometrie durch einen Bediener.

In einem weiteren Aspekt existiert ein medizinisches Instrumentenset mit mindestens einem erfindungsgemäßen Werkzeug und einem Handstück mit einem Antrieb und einem Werkzeugfutter, wobei das Handstück vorgesehen und ausgebildet ist, das Werkzeug in dem Werkzeugfutter aufzunehmen, dadurch gekennzeichnet, dass das Handstück eine Rast- und Antriebseinheit aufweist, die das Werkzeug in einem in das Handstück eingesetzten Zustand axial sichert und über die ein Antriebsdrehmoment auf das Werkzeug übertragen wird.

In anderen Worten ist ein medizinisches Instrumentenset offenbart, das zumindest ein offenbarungsgemäßes Werkzeug und ein Handstück beinhaltet. Das Handstück ist mit einem Antrieb versehen, wobei es sich bei dem Antrieb vorzugsweise um einen Elektromotor oder eine Druckluftturbine handelt. Das Handstück weist die Rast- und Antriebseinheit auf, die vorgesehen und ausgebildet ist, in die Schaftgeometrie des Werkzeugs einzurasten. In einem eingerasteten Zustand ist das Werkzeug axial über die Rast- und Antriebseinheit gesichert und es wird das von dem Antrieb erzeugte Drehmoment über die Rast- und Antriebseinheit auf das Werkzeug übertragen.

In einem weiteren bevorzugten Aspekt weist die Rast- und Antriebseinheit des Handstücks des medizinischen Instrumentensets Tonnen auf.

In anderen Worten ist die Rast- und Antriebseinheit mit Rastelementen/Tonnen ausgestattet, die in die Schaftgeometrie des Werkzeugs einrasten und über welche sowohl das Drehmoment übertragen wird, als auch das Werkzeugs axial in dem Werkzeug gesichert wird. Durch die flächige Abstützung der Tonnen in den muldenförmigen Einbuchtungen kann ein hohes Drehmoment von der Rast- und Antriebseinheit auf das Werkzeug übertragen werden. Die Tonnen sind dabei vorzugsweise aus einem keramischen Material gefertigt. Dies ermöglicht eine gute Drehmomentübertragung bei geringer Eingriffstiefe der Tonnen in die muldenförmigen Einbuchtungen.

In einem weiteren bevorzugten Aspekt entspricht die Anzahl der Tonnen in dem Handstück des medizinischen Instrumentensets der Anzahl der muldenförmigen Einbuchtungen.

In anderen Worten sind die Rast- und Antriebseinheit des Handstücks und das Werkzeug dergestalt aufeinander abgestimmt, dass jede Tonne der Rast- und Antriebseinheit in eine der muldenförmigen Einbuchtungen der Schaftgeometrie des Werkzeugs einrastet. Dies gewährleistet eine gleichmäßige Drehmomentübertragung von der Rast- und Antriebseinheit auf das Werkzeug.

In einem weiteren bevorzugten Aspekt beinhaltet das Handstück des medizinischen Instrumentensets ein Nachfahrelement, welches eine Negativgeometrie des Ausrichtungsabschnitts aufweist und vorgesehen und ausgebildet ist, den Ausrichtungsabschnitt des Werkzeugs in einem eingerasteten Zustand aufzunehmen.

In anderen Worten ist das Handstück des medizinischen Instrumentensets mit einem Nachfahrelement ausgestattet, das mittels einer Feder in distaler Richtung mit einer Federkraft beaufschlagt wird. Das Nachfahrelement weist an einer distalen Stirnseite eine Geometrie auf, die der Negativgeometrie des Ausrichtungsabschnitts des Werkzeugs entspricht und das Werkzeug mit dem Ausrichtungsabschnitt aufnimmt.

In einem weiteren bevorzugten Aspekt ist das Nachfahrelement des Handstücks des medizinischen Instrumentensets so konfiguriert, dass das Nachfahrelement das Werkzeug bei einem Einsetzen des Werkzeugs in das Handstück so vorbestimmt orientiert/positioniert, dass die Tonnen in die muldenförmigen Einbuchtungen eingreifen.

In anderen Worten gleitet bei einem Einsetzen des Werkzeugs in das Handstück der Ausrichtungsabschnitt des Werkzeugs in die Negativgeometrie des Nachfahrelements. Das Nachfahrelement ist radial verdrehsicher in dem Handstück gelagert und radial so positioniert, dass die muldenförmigen Einbuchtungen der Schaftgeometrie, wenn das Werkzeug mit dem Ausrichtungsabschnitt in dem Nachfahrelement liegt, radial entsprechend den Tonnen der Rast- und Antriebseinheit des Handstücks orientiert sind.

In nochmals anderen Worten sorgt das Nachfahrelement dafür, dass die muldenförmigen Einbuchtungen des Werkzeugs vor dem Einrasten in radialer Richtung deckungsgleich zu den Tonnen der Rast- und Antriebseinheit des Handstücks orientiert sind.

Durch die radial lagerichtige Orientierung des Werkzeugs bzw. der Schaftgeometrie zu der Rast- und Antriebseinheit vor dem Einrasten ist gewährleistet, dass das Werkzeug vollständig einrasten kann und so Beschädigungen an dem Werkzeug oder an dem Handstück vermieden werden können. Zusätzlich erhöht es den Komfort des Bedieners, der keinen Abgleich zwischen der radialen Orientierung des Werkzeugs und des Handstücks vornehmen muss.

In einem weiteren bevorzugten Aspekt beinhaltet das Handstück des medizinischen Instrumentensets eine Identifikationseinheit, welche proximal von der Handstücköffnung und distal von der Rast- und Antriebseinheit in dem Schaft des Handstücks ausgebildet/angebracht ist.

In anderen Worten ist in dem Schaft des Handstücks die Identifikationseinheit ausgebildet, die bei dem Einsetzen des Werkzeugs die auf dem Identifizierungselement des Werkzeugs gespeicherten Daten ausließt. Die Identifikationseinheit ist dabei zwischen den Lagerstellen des Werkzeugs in dem Schaft des Handstücks ausgebildet.

In nochmals anderen Worten ist die Identifikationseinheit in einem distalen Abschnitt des Handstücks ausgebildet und detektiert vorzugsweise während des Einsetzvorgangs des Werkzeugs in das Handstück die auf dem Identifizierungselement hinterlegten Daten.

Weiterhin kann die Identifikationseinheit ausgebildet sein, bei einem Herausziehvorgang des Werkzeugs aus dem Handstück Daten auf das Identifizierungselement zu schreiben.

Auch kann die Identifikationseinheit so in dem Handstück angeordnet sein, dass während des gesamten Zeitraums, in dem das Werkzeug in das Handstück eingerastet ist, das Identifizierungselement gelesen und beschrieben werden kann.

### Kurzbeschreibung der Figuren

Die Offenbarung wird nachstehend unter Bezugnahme auf die beigefügten Zeichnungen näher beschrieben.
Fig. 1 ist eine Darstellung eines medizinischen Instrumentensets mit einem erfindungsgemäßen Werkzeug und einem Handstück in einem Zustand, in dem das Werkzeug nicht in das Handstück eingesetzt ist.
Fig. 2 ist eine Darstellung eines Schnittes des medizinischen Instrumentensets mit dem erfindungsgemäßen Werkzeug und dem Handstück in einem Zustand, in dem das Werkzeug in das Handstück eingesetzt ist.
Fig. 3 sind Darstellungen des erfindungsgemäßen Werkzeugs mit einer Schaftgeometrie und einem Identifizierungselement in einer Hülse der ersten Ausführungsform, die mit einem Ausrichtungsabschnitt versehen ist.
Fig. 4 ist eine Darstellung eines Schnittes des erfindungsgemäßen Werkzeugs und des Handstücks in einem Zustand, in dem das Werkzeug in das Handstück eingeführt wird und der Ausrichtungsabschnitt des Werkzeugs nicht in Kontakt mit einem Nachfahrelement des Handstücks steht.
Fig. 5 ist eine Darstellung eines Schnittes des erfindungsgemäßen Werkzeugs und des Handstücks in einem Zustand, in dem das Werkzeug in das Handstück eingeführt ist und der Ausrichtungsabschnitt des Werkzeugs in Kontakt mit dem Nachfahrelement des Handstücks steht.
Fig. 6 ist eine vergrößerte Darstellung eines Schnittes des erfindungsgemäßen Werkzeugs und des Handstücks in einem Zustand, in dem das Werkzeug in das Handstück eingeführt ist und der Ausrichtungsabschnitt des Werkzeugs in Kontakt mit dem Nachfahrelement des Handstücks steht.
Fig. 7 ist eine Darstellung eines Schnittes der Schaftgeometrie, des Identifizierungselements und der Hülse mit Ausrichtungsabschnitt des erfindungsgemäßen Werkzeugs in einem eingerasteten Zustand des Werkzeugs in dem Handstück.
Fig. 8 ist eine Darstellung eines Schnittes des erfindungsgemäßen Werkzeugs und des Handstücks in einem eingerasteten Zustand.
Fig. 9 ist eine Darstellung einer Frontalansicht des Handstücks mit einem Schnitt quer durch das Nachfahrelement.
Fig. 10 ist eine Darstellung einer weiteren Frontalansicht des Handstücks mit einem Schnitt quer durch Tonnen.
Fig. 11 ist eine Darstellung einer zweiten Ausführungsform der Hülse, verbunden mit dem Werkzeugschaft.
Fig. 12 ist eine Darstellung einer dritten Ausführungsform der Hülse, verbunden mit dem Werkzeugschaft.

Nachstehend werden Ausführungsbeispiele der vorliegenden Offenbarung auf der Basis der zugehörigen Figuren beschrieben. In den Figuren bezeichnen gleiche Bezugszeichen gleiche oder zumindest äquivalente Teile und Komponenten. Zweckmäßig wird insoweit auf eine mehrfach redundante Beschreibung solcher Teile und Komponenten verzichtet.

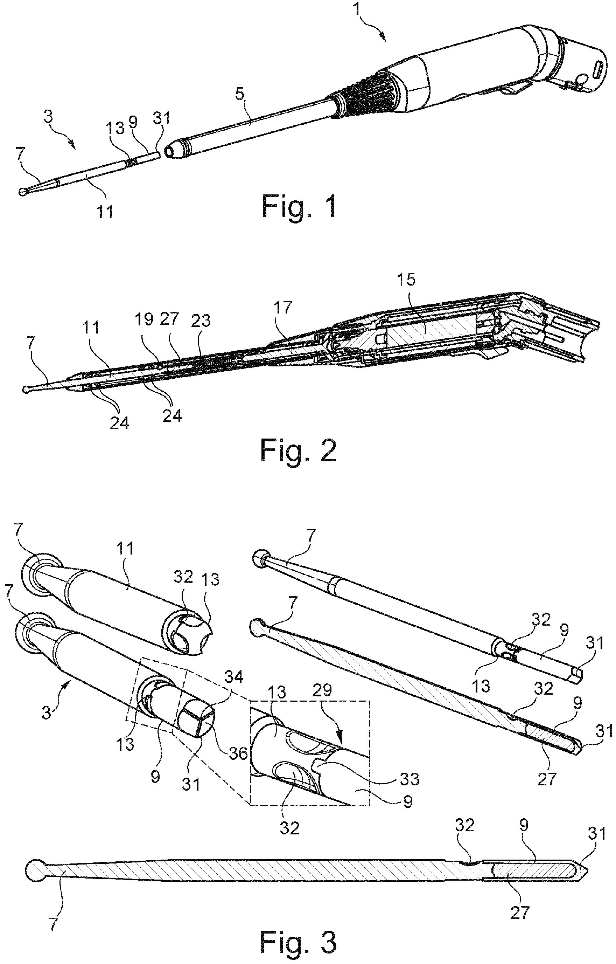

Fig. 1 zeigt ein medizinisches Instrumentenset mit einem Handstück 1 und einem erfindungsgemäßen Werkzeug 3, das dafür vorgesehen und ausgebildet ist, in einen Schaft 5 des Handstücks 1 eingesetzt zu werden. Das Werkzeug 3 weist ein distales Arbeitsende 7 auf, das die eigentliche Funktion des Werkzeugs 3 (beispielsweise Fräsen, Bohren, Schleifen oder Glätten, etc.) ausbildet. An der dem Arbeitsende 7 gegenüberliegenden proximalen Seite des Werkzeugs 3 ist eine (separat gefertigte) Hülse 9 vorgesehen, die kraft- und/oder form- und/oder stoffschlüssig mit einem Werkzeugschaft 11 verbunden ist. Der Werkzeugschaft 11 verbindet das distale Arbeitsende 7 mit der proximalen Hülse 9 und ist vorzugsweise einstückig, insbesondere stoffeinstückig mit dem Arbeitsende 7 ausgebildet. Auf/in dem Werkzeugschaft 11 ist eine (radial nach außen exponierte) Schaftgeometrie 13 ausgebildet.

Fig. 2 zeigt einen Schnitt durch das medizinische Instrumentenset mit dem Handstück 1 und dem erfindungsgemäßen Werkzeug 3 in einem Zustand, in dem das Werkzeug 3 in das Handstück 1 eingesetzt/eingerastet ist.

Das Handstück 1 beinhaltet vorliegend einen Antrieb 15, der vorgesehen und ausgebildet ist, das Werkzeug 1 (rotatorisch) anzutreiben. Der Antrieb 15 ist vorzugsweise als eine beispielsweise mit Druckluft angetriebene Turbine oder als ein Elektromotor ausgebildet. Der Antrieb 15 übt ein Antriebsmoment auf eine vorzugsweise wälz- oder gleitgelagerte Antriebswelle 17 aus. An dem dem Antrieb 15 gegenüberliegenden Endabschnitt der Antriebswelle 17 ist eine Rast- und Antriebseinheit 19 (Werkzeugaufnahmefutter) ausgebildet.

Vorzugsweise ist in der Rast- und Antriebseinheit 19 ein Nachfahrelement 21 ausgebildet, das durch eine Feder 23 in Richtung hin zur Antriebswelle 17 mit einer Federkraft beaufschlagt wird. Das Nachfahrelement 21 ist vorzugsweise verdrehsicher/verdrehfest um seine Längsachse in der Rast- und Antriebseinheit 19 gelagert. Die Schaftgeometrie 13 des Werkzeugs 3 ist in die Rast- und Antriebseinheit 19 des Handstücks 1 eingerastet, sodass das Antriebsmoment des Antriebs 15 auf das Werkzeug 3 übertragen werden kann. Das Werkzeug 3 ist in dem eingerasteten Zustand in dem Schaft 5 mittels Lager 24 gelagert, sodass das Werkzeug 3 in dem Schaft 5 verlustarm um seine Längsachse rotieren kann. Vorzugsweise zwischen den Lagern 24 ist in dem Schaft eine Identifikationseinheit 25 ausgebildet. Die Identifikationseinheit 25 identifiziert vorzugsweise bei einem Einschieben des Werkzeugs 3 in den Schaft 5 des Handstücks 1 das Werkzeug 3 anhand eines Identifizierungselements 27, das vorzugsweise in der Hülse 9 ausgebildet/aufgenommen ist.

Das Identifizierungselement 27 ist vorzugsweise als eine drahtlos ausles- und/oder beschreibbare Speichereinheit ausgebildet, welche Informationen speichert, die eine Erkennung des Werkzeugs bzw. des Arbeitsendes des Werkzeugs gewährleisten. Zusätzlich können weitere, durch einen Benutzer festgelegte Daten und Informationen auf der Speichereinheit gespeichert sein. Vorzugsweise werden anwendungsbezogene und/oder prozessbezogene Daten wie eine Artikelnummer, eine grafische Darstellung des Werkzeugs, eine LOT-Angabe, ein Mindesthaltbarkeitsdatum, Einsatzparameter und dergleichen auf der Speichereinheit gespeichert.

Das Identifizierungselement 27 ist vorzugsweise via Bluetooth und/oder NFC oder dergleichen drahtlos ausles- und/oder beschreibbar.

Fig. 3 zeigt verschiedene Ansichten und Schnitte des erfindungsgemäßen Werkzeugs 3. Das Werkzeug beinhaltet demnach das distale Arbeitsende 7, den Werkzeugschaft 11 mit der Schaftgeometrie 13, die proximale Hülse 9 und das Identifizierungselement 27 in der Hülse 9 sowie eine zusätzliche (Drehausrichtungs-) Positionierhilfe 31, welche bevorzugt am proximalen Endabschnitt des Hülse 9 angeordnet/in die Hülse 9 eingearbeitet ist. Schließlich sind am proximalen Endabschnitt des Werkzeugschafts 11 und am distalen Endabschnitt der Hülse jeweils ein miteinander in Wirkeingriff bringbares Ausrichtungsprofil 29 angeordnet/ausgebildet, wodurch die Hülse 9 bei deren Montage am Werkzeugschaft 11 eine vordefinierte Relativdrehposition zum Werzeugschaft 11 einnimmt und somit die proximale (Drehausrichtungs-) Positionierhilfe bzw. Ausrichtungsabschnitt 31 ebenfalls eine vordefinierte Relativdrehposition zum Werzeugschaft 11 erhält.

Das Arbeitsende 7 ist, wie vorstehend bereits angedeutet, vorzugsweise einstückig mit dem Werkzeugschaft 11 ausgebildet.

Auf der Mantelfläche des Werkzeugschafts 11 ist die Schaftgeometrie 13 ausgebildet. Die Schaftgeometrie 13 ist vorgesehen und ausgebildet, ein Drehmoment, das über die Rast- und Antriebseinheit 19 des Handstücks1 in das Werkzeug 3 eingeleitet wird, zu übertragen und gleichzeitig auch das Werkzeug 3 axial in dem Handstück 1 zu sichern. Die Schaftgeometrie 13 ist offenbarungsgemäß als mulden- oder badewannenförmige Vertiefung/Einbuchtung 32 in der Mantelfläche des Werkzeugschafts 11 ausgebildet. Vorzugsweise ist die Schaftgeometrie 13 als mehrere, gleichmäßig über den Umfang des Werkzeugs 3 verteilte muldenförmige Einbuchtungen 32 ausgebildet.

Proximal zu der Schaftgeometrie 13 ist die Hülse 9 vorgesehen oder ausgebildet. Die Hülse 9 ist vorzugsweise auf den Werkzeugschaft 11 geklebt, ultraschallgeschweißt, aufgeschrumpft, aufgelötet oder aufgepresst. Die von dem Arbeitsende 7 abgewandte (proximale) Stirnseite der Hülse 9 bildet die Positionierhilfe (Ausrichtungsabschnitt) 31 und ist vorzugsweise pyramidenförmig ausgebildet, wobei die Spitze 34 der Pyramide auf der Mittelfaser des Werkzeugs liegt und die Pyramidenseiten 36 radial so ausgerichtet sind, dass jede Pyramidenseite 36 einer der muldenförmigen Einbuchtungen 32 zugewandt ist.

Diese vorbestimmte/lagerichtige Orientierung des Ausrichtungsabschnitts 31 zu der Schaftgeometrie 13 ist über die Positionierungshilfe 29 festgelegt. Die Positionierungshilfe 29 ist vorzugsweise als Zapfen 33 oder als Stift an der Hülse 9 ausgebildet, der in eine entsprechende Negativform in dem Werkzeugschaft 11 bzw. der Schaftgeometrie 13 passt, und damit die Hülse 9 und den Ausrichtungsabschnitt 31 radial lagerichtig zu der Schaftgeometrie 13 orientiert.

Die Hülse 9 ist vorgesehen und ausgebildet, das Identifizierungselement 27 in sich aufzunehmen. Vorzugsweise hat das Identifizierungselement 27 einen kleineren Durchmesser als der Werkzeugschaft 11. Insbesondere vorzugsweise ist der Durchmesser des Identifizierungselements 27 kleiner gleich 1,4 mm. Weiterhin vorzugsweise beträgt die axiale Länge des Identifizierungselements 27 8 mm. Vorzugsweise ist die Hülse 9 so ausgebildet, dass in einem Zustand, in dem die Hülse 9 und der Werkzeugschaft 11 verbunden sind, das Identifizierungselement 27 formschlüssig in der Hülse 9 befestigt ist.

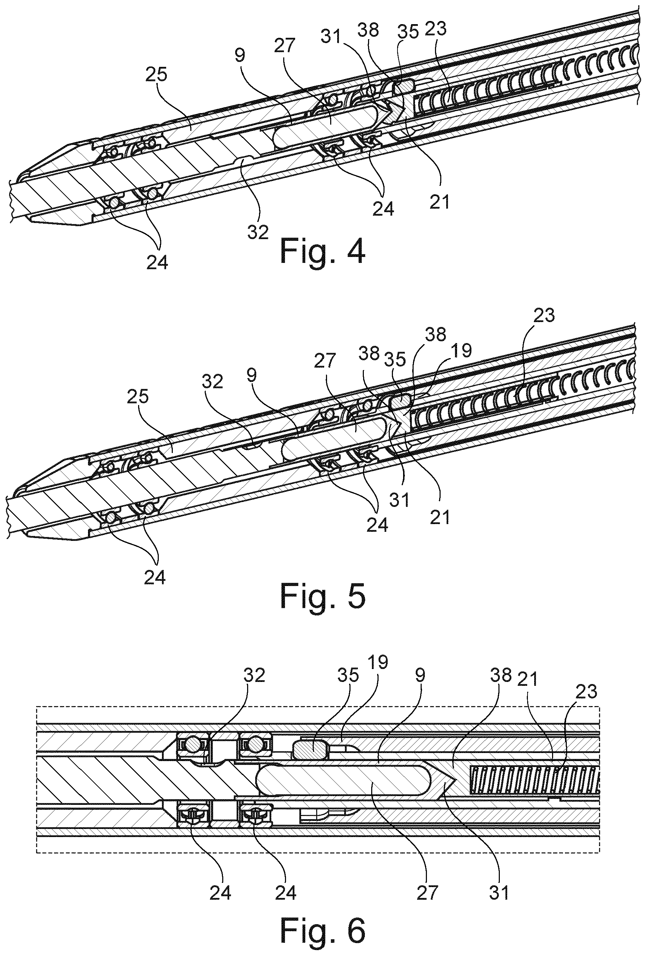

Nachfolgend wird das Einsetzen des Werkzeugs 3 in das Handstück 1 anhand von Fig. 4 bis Fig. 8 beschrieben.

Fig. 4 zeigt einen Schnitt durch das medizinische Instrumentenset in einem Zustand, in dem das Werkzeug 3 so in den Schaft 5 des Handstücks 1 eingeführt ist, dass die Schaftgeometrie 13 nicht lagerichtig zu der Rast- und Antriebseinheit 19 ausgerichtet ist. In anderen Worten ist das Werkzeug 3 so ausgerichtet, dass die muldenförmigen Einbuchtungen der Schaftgeometrie 13 radial nicht in Deckung mit den Rastelementen/Tonnen 35 der Rast- und Antriebseinheit 19 stehen. Der Ausrichtungsabschnitt 31 ist nicht in Kontakt mit dem Nachfahrelement 21. Das Nachfahrelement 21 weist an einer dem Werkzeug 3 zugewandten Stirnseite eine Ausrichtungsgeometrie 38 auf, die der Negativgeometrie des Ausrichtungsabschnitts 31 entspricht. Diese Ausrichtungsgeometrie 38 ist axial verdrehfest so ausgerichtet, dass die Flächen der Ausrichtungsgeometrie 38 in ihrer radialen Anordnung der radialen Anordnung der Rastelemente/Tonnen 35 der Rast- und Antriebseinheit 19 entsprechen.

Anders ausgedrückt ist die Ausrichtungsgeometrie 38 des Nachfahrelements 21 eine negative Pyramidenform, die in einem Schlüssel-Schlossprinzip zu der Pyramidenform des Ausrichtungsabschnitts 31 des Werkzeugs 3 passt. Die Seitenflächen der negativen Pyramidenform sind radial so ausgerichtet, dass ihre Ausrichtung der radialen Ausrichtung der Rastelemente/Tonnen 35 der Rast- und Antriebseinheit 19 entspricht.

Während des Einführens des Werkzeugs 3 in den Schaft 5 des Handstücks 1 passiert das in der Hülse 9 positionierte Identifizierungselement 27 die in dem Schaft angeordnete Identifikationseinheit 25, die drahtlos die auf dem Identifizierungselement 27 gespeicherten Informationen ausließt.

Fig. 5 zeigt einen Schnitt durch das medizinische Instrumentenset in einem Zustand, in dem das Werkzeug 3 etwas weiter als in Fig. 4 in den Schaft 5 des Handstücks 1 eingeführt ist. Während des Einführens kommt der Ausrichtungsabschnitt 31 mit dem Nachfahrelement 21 an dessen Stirnseite in Kontakt. Da das Nachfahrelement 21 radial verdrehfest ausgebildet ist, dreht sich das Werkzeug so lange um seine Längsachse, bis die Seitenflächen der Pyramide des Ausrichtungsabschnitts 31 entsprechend den Seitenflächen der negativen Pyramide der Ausrichtungsgeometrie 38 des Nachfahrelements 21 radial ausgerichtet sind. Der Ausrichtungsabschnitt 31 des Werkzeugs und die Ausrichtgeometrie 38 des Nachfahrelements 21 stehen dann in flächigem Kontakt und die muldenförmigen Einbuchtungen 32 der Schaftgeometrie 13 sind radial entsprechend der radialen Anordnung der Rastelemente/Tonnen 35 der Rast- und Antriebseinheit 19 ausgerichtet.

Fig. 6 zeigt einen Schnitt durch das medizinische Instrumentenset in einem Zustand, in dem das Werkzeug 3 etwas weiter als in Fig. 5 in den Schaft 5 des Handstücks 1 eingeführt ist. Die Feder 23, die das Nachfahrelement 21 in Richtung der Antriebswelle 17 mit der Federkraft beaufschlagt, wird durch den Bediener durch das einschieben des Werkzeugs 3 in den Schaft 5 gestaucht.

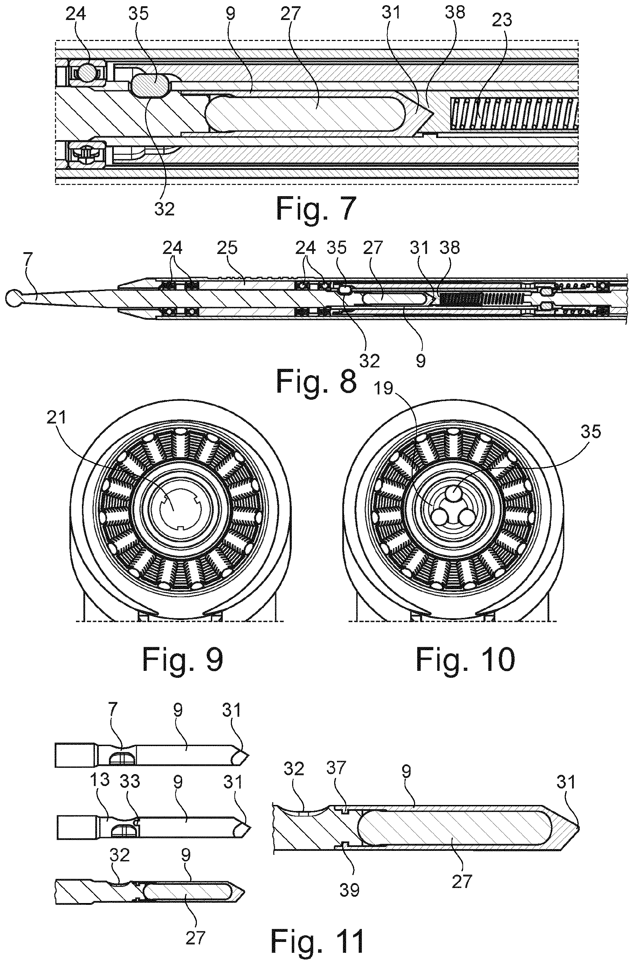

Fig. 7 zeigt einen Schnitt durch das medizinische Instrumentenset in einem Zustand, in dem das Werkzeug 3 so weit in den Schaft 5 des Handstücks 1 eingeführt ist, dass das Werkzeug 3 eingerastet ist. In anderen Worten befindet sich in jeder der muldenförmigen Einbuchtungen 32 der Schaftgeometrie 13 des Werkzeuges 3 in einem eingerasteten Zustand ein Rastelement/eine Tonne 35 der Rast- und Antriebseinheit 19. Durch das Eingreifen der Rast- und Antriebseinheit 19 des Handstücks in die Schaftgeometrie 13 des Werkzeugs 3 ist Drehmomenteinleitung von dem Antrieb 15 des Handstücks 1 in das Werkzeug 3 sowie die axiale Sicherung des Werkzeugs 3 im Handstück 1 umgesetzt.

Auch in Fig. 8 ist ein Schnitt durch das medizinische Instrumentenset in einem Zustand dargestellt, in dem das Werkzeug 3 in dem Handstück 1 eingerastet ist. Die Feder 23 ist hierbei gestaucht und das Werkzeug ist drehbar um die Mittelfaser des Werkzeugs in den Lagern 24 gelagert.

Fig. 9 ist eine Frontalansicht des Handstücks 1, in der das Nachfahrelement 21 geschnitten dargestellt ist, insbesondere um eine Verdrehsicherung zischen dem Nachfahrelement 21 und der Rast- und Antriebseinheit 19 darzustellen.

Auch in Fig. 10 ist eine Frontalansicht des Handstücks 1 dargestellt. Explizit ist ein Schnitt dargestellt, der zeigt wie die Rastelemente/Tonnen 35 der Rast- und Antriebseinheit 19 in die muldenförmigen Einbuchtungen 32 der Schaftgeometrie 13 eingreifen. Das Antriebsmoment wird über die Rastelemente/Tonnen 35 insbesondere auf die Flanken der muldenförmigen Einbuchtungen 32 der Schaftgeometrie 13 übertragen.

Fig. 11 zeigt eine alternative Ausführungsform der Befestigung der Hülse 9 an dem Werkzeugschaft 11. Die Hülse 9 weist an einem Öffnungsabschnitt der Hülse 9 eine zur Mittelfaser der Hülse 9 hin orientierte Rastkontur 37 auf, die in einem montierten Zustand in einen vorzugsweise umlaufenden Einstich 39 an dem Werkzeugschaft 11 eingreift. Die vorbestimmte Orientierung ist auch in dieser Ausführungsform vorzugsweise mittels des Zapfens 33 sichergestellt.

Alternativ kann über einen asymmetrischen Einstich 39 und eine entsprechend asymmetrisch ausgebildete Rastkontur 37 eine vorbestimmte Orientierung der Hülse 9 zu dem Werkzeugschaft 11 definiert sein.

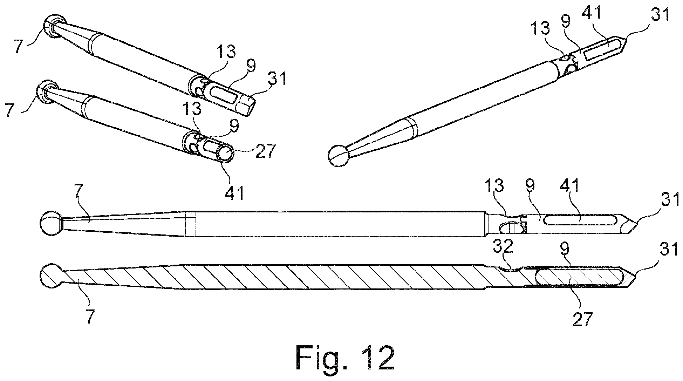

Fig. 12 zeigt eine weiter alternative Ausführungsform der Hülse 9. Die Hülse 9 besteht in dieser Ausführungsform vorzugsweise aus Metall und weist radial um den Umfang der Hülse 9 verteilte Schlitze 41 auf. Die Schlitze 41 sind vorzugsweise mit einer Vergussmasse oder mit Kunstoffeinlegeteilen verschlossen, um eine gute Ansprechbarkeit des Identifizierungselements 27 zu gewährleisten. Vorzugsweise beträgt die Anzahl der Schlitze 41 in der Hülse 9 zwischen 1 und 10, insbesondere vorzugsweise zwischen 1 und 3.

### Bezugszeichenliste

- 1: Handstück
- 3: Werkzeug
- 5: Schaft
- 7: Arbeitsende
- 9: Hülse
- 11: Werkzeugschaft
- 13: Schaftgeometrie
- 15: Antrieb
- 17: Antriebswelle
- 19: Rast- und Antriebseinheit
- 21: Nachfahrelement
- 23: Feder
- 24: Lager
- 25: Identifikationseinheit
- 27: Identifizierungselement
- 29: Positionierhilfe
- 31: Ausrichtungsabschnitt
- 32: muldenförmige Einbuchtung
- 33: Zapfen
- 34: Spitze des pyramidenförmigen Ausrichtungsabschnitts
- 35: Rastelement/Tonne
- 36: Seitenfläche des pyramidenförmigen Ausrichtungsabschnitts
- 37: Rastkontur
- 38: Ausrichtungsgeometrie
- 39: Einstich
- 41: Schlitz

## Patentansprüche

1. Medizinisches Werkzeug (3) mit einem Werkzeugschaft (11), der in einem Schaftbereich eine Schaftgeometrie (13) zur Drehmomenteinleitung und Axialsicherung aufweist, wobei die Schaftgeometrie (13) zur Drehmomenteinleitung und auch Axialsicherung ausschließlich eine oder ausschließlich mehrere muldenförmige Einbuchtung(en) (32) in der äußeren Mantelfläche des Werkzeugschafts (11) ist, wobei das Werkzeug (3) einen proximal zu der Schaftgeometrie (13) angeordneten Ausrichtungsabschnitt (31) beinhaltet, wobei das Werkzeug (3) ein Identifizierungselement (27) aufweist, **dadurch gekennzeichnet, dass** das Identifizierungselement (27) in einer Hülse (9) angeordnet ist, die den Ausrichtungsabschnitt (31) beinhaltet, wobei der Ausrichtungsabschnitt (31) eine pyramidenförmige Geometrie aufweist.

2. Werkzeug (3) nach Anspruch 1, **dadurch gekennzeichnet, dass** die Schaftgeometrie (13) aus mehreren, gleichmäßig über dem Umfang des Werkzeugs (3) verteilten, muldenförmigen Einbuchtungen (32) besteht.

3. Werkzeug (3) nach Anspruch 1 oder 2, **dadurch gekennzeichnet, dass** die Anzahl der muldenförmigen Einbuchtungen (32) ein ganzzahliges Vielfaches der Anzahl der Pyramidenseiten (36) ist und vorzugsweise eine Anzahl von Pyramidenseiten (36) einer Anzahl von muldenförmigen Einbuchtungen (32) entspricht.

4. Werkzeug (3) nach einem der Ansprüche 1 bis 3, **dadurch gekennzeichnet, dass** die Hülse (9) einen Innenraum aufweist, der vorgesehen und ausgebildet ist, ein Identifizierungselement (27) von vorzugsweise 1,4 mm x 8 mm aufzunehmen.

5. Werkzeug (3) nach einem der Ansprüche 1 bis 4, **dadurch gekennzeichnet, dass** die Hülse (9) einstückig, insbesondere stoffeinstückig, ausgebildet ist.

6. Werkzeug (3) nach einem der Ansprüche 1 bis 5, **dadurch gekennzeichnet, dass** die Hülse (9) kraft- und/ oder formschlüssig mit dem Werkzeugschaft (11) verbunden ist.

7. Werkzeug (3) nach einem der Ansprüche 1 bis 6, **dadurch gekennzeichnet, dass** der Ausrichtungsabschnitt (31) in Relation zu der Schaftgeometrie (13) vorbestimmt orientiert ist und die vorbestimmte Orientierung über eine Positionierhilfe (29) zwischen dem Ausrichtungsabschnitt (31) und der Schaftgeometrie (13) festgelegt wird.

8. Werkzeug (3) nach einem der Ansprüche 1 bis 7, **dadurch gekennzeichnet, dass** die Hülse (9) aus Kunststoff ausgebildet ist oder dass in der Hülse (9) Schlitze vorgesehen sind, welche optional mit einer Vergussmasse oder Kunststoffeinlegeteilen verschlossen sind.

9. Medizinisches Instrumentenset mit mindestens einem Werkzeug (3) nach einem der Ansprüche 1 bis 8 und einem Handstück (1) mit einem Antrieb (15) und einem Werkzeugfutter, wobei das Handstück (1) vorgesehen und ausgebildet ist, das Werkzeug (3) aufzunehmen, **dadurch gekennzeichnet, dass** das Handstück (1) eine Rast -und Antriebseinheit (19) aufweist, die das Werkzeug (3) in einem in das Handstück (1) eingesetzten Zustand axial sichert und über die ein Antriebsdrehmoment auf das Werkzeug (3) übertragen wird.

10. Medizinisches Instrumentenset nach Anspruch 9, **dadurch gekennzeichnet, dass** die Rast -und Antriebseinheit (19) Tonnen (35) aufweist.

11. Medizinisches Instrumentenset nach Anspruch 10, **dadurch gekennzeichnet, dass** die Anzahl der Tonnen (35) in dem Handstück (1) der Anzahl der muldenförmigen Einbuchtungen (32) entspricht.

12. Medizinisches Instrumentenset nach einem der Ansprüche 9 bis 11, **dadurch gekennzeichnet, dass** das Handstück (1) ein Nachfahrelement (21) beinhaltet, welches eine Negativgeometrie des Ausrichtungsabschnitts aufweist und vorgesehen und ausgebildet ist, den Ausrichtungsabschnitt (31) des Werkzeugs (3) in einem eingerasteten Zustand aufzunehmen.

13. Medizinisches Instrumentenset nach Anspruch 12, **dadurch gekennzeichnet, dass** das Nachfahrelement (21) das Werkzeug (3) bei einem Einsetzen des Werkzeugs (3) in das Handstück (1) so vorbestimmt orientiert/positioniert, dass die Tonnen (35) in die muldenförmigen Einbuchtungen (32) eingreifen.

14. Medizinisches Instrumentenset nach einem der Ansprüche 9 bis 13, **dadurch gekennzeichnet, dass** das Handstück (1) eine Identifikationseinheit (25) beinhaltet, welche proximal von der Handstücköffnung und distal von der Rast- und Antriebseinheit (19) in dem Schaft (5) des Handstücks (1) ausgebildet/angebracht ist.

## Claims

1. A medical tool (3) comprising a tool shaft (11) that has, in a shaft region, a shaft geometry (13) for introducing torque and axially securing, wherein the shaft geometry (13) for introducing torque and also axially securing is exclusively one or exclusively a plurality of trough-shaped indentation(s) (32) in the outer lateral surface of the tool shaft (11), wherein the tool (3) includes an orientation portion (31) arranged proximally to the shaft geometry (13), wherein the tool (3) comprises an identification element (27), **characterized in that** the identification element (27) is arranged in a sleeve (9) containing the orientation portion (31), wherein the orientation portion (31) preferably has a pyramid-shaped geometry.

2. The tool (3) according to claim 1, **characterized in that** the shaft geometry (13) consists of several trough-shaped indentations (32) distributed evenly over the circumference of the tool (3).

3. The tool (3) according to claim 1 or 2, **characterized in that** the number of the trough-shaped indentations (32) is an integer multiple of the number of the pyramid sides (36), and preferably a number of pyramid sides (36) corresponds to a number of trough-shaped indentations (32).

4. The tool (3) according to one of the claims 1 to 3, **characterized in that** the sleeve (9) has an inner space which is provided and configured to receive an identification element (27) of preferably 1.4 mm x 8 mm.

5. The tool (3) according to one of claims 1 to 4, **characterized in that** the sleeve (9) is formed in one piece, in particular in one piece of material.

6. The tool (3) according to one of claims 1 to 5, **characterized in that** the sleeve (9) is connected to the tool shaft (11) in a force-fitting and/or form-fitting manner.

7. The tool (3) according to one of claims 1 to 6, **characterized in that** the orientation portion (31) is oriented in a predetermined manner in relation to the shaft geometry (13) and the predetermined orientation is fixed via a positioning aid (29) between the orientation portion (31) and the shaft geometry (13).

8. The tool (3) according to one of claims 1 to 7, **characterized in that** the sleeve (9) is made of plastic or that slits are provided in the sleeve (9), which are optionally closed with a casting compound or plastic inserts.

9. A medical instrument set comprising at least one tool (3) according to one of claims 1 to 8 and a handpiece (1) having a drive (15) and a tool chuck, wherein the handpiece (1) is provided and configured to receive the tool (3), **characterized in that** the handpiece (1) comprises a latching and drive unit (19) which axially secures the tool (3) in a state inserted in the handpiece (1) and via which a drive torque is transmitted to the tool (3).

10. The medical instrument set according to claim 9, **characterized in that** the latching and drive unit (19) comprises barrels (35).

11. The medical instrument set according to claim 10, **characterized in that** the number of barrels (35) in the handpiece (1) corresponds to the number of trough-shaped indentations (32).

12. The medical instrument set according to one of claims 9 to 11, **characterized in that** the handpiece (1) includes a follow element (21) having a negative geometry of the orientation portion and being provided and configured to receive the orientation portion (31) of the tool (3) in a latched state.

13. The medical instrument set according to claim 12, **characterized in that** the follow element (21) orients/positions the tool (3) in a predetermined manner when the tool (3) is inserted into the handpiece (1) such that the barrels (35) engage the trough-shaped indentations (32).

14. The medical instrument set according to one of claims 9 to 13, **characterized in that** the handpiece (1) comprises an identification unit (25) configured/mounted proximally of the handpiece opening and distally of the latching and drive unit (19) in the shaft (5) of the handpiece (1).

## Revendications

1. Outil médical (3) avec une tige d'outil (11) qui présente dans une zone de tige une géométrie de tige (13) pour l'introduction de couple et la fixation axiale, dans lequel la géométrie de tige (13) pour l'introduction de couple et aussi la fixation axiale est exclusivement un ou exclusivement plusieurs creux (32) en forme de cavité dans la surface d'enveloppe extérieure de la tige d'outil (11), dans lequel l'outil (3) contient une partie d'alignement (31) agencée de manière proximale à la géométrie de tige (13), dans lequel l'outil (3) présente un élément d'identification (27), **caractérisé en ce que** l'élément d'identification (27) est agencé dans une douille (9) qui contient la partie d'alignement (31), dans lequel la partie d'alignement (31) présente une géométrie en forme de pyramide.

2. Outil (3) selon la revendication 1, **caractérisé en ce que** la géométrie de tige (13) se compose de plusieurs creux (32) en forme de cavité, répartis uniformément sur la périphérie de l'outil (3).

3. Outil (3) selon la revendication 1 ou 2, **caractérisé en ce que** le nombre de creux (32) en forme de cavité est un multiple entier du nombre de côtés de la pyramide (36) et de préférence un nombre de côtés de la pyramide (36) correspond à un nombre de creux (32) en forme de cavité.

4. Outil (3) selon l'une quelconque des revendications 1 à 3, **caractérisé en ce que** la douille (9) présente un espace intérieur qui est prévu et réalisé afin de recevoir un élément d'identification (27) de préférence de 1,4 mm x 8 mm.

5. Outil (3) selon l'une quelconque des revendications 1 à 4, **caractérisé en ce que** la douille (9) est réalisée d'un seul tenant, en particulier en un seul matériau.

6. Outil (3) selon l'une quelconque des revendications 1 à 5, **caractérisé en ce que** la douille (9) est reliée à force et/ou par complémentarité de formes à la tige d'outil (11).

7. Outil (3) selon l'une quelconque des revendications 1 à 6, **caractérisé en ce que** la partie d'alignement (31) est orientée de manière prédéterminée par rapport à la géométrie de tige (13) et l'orientation prédéterminée est fixée par le biais d'une aide au positionnement (29) entre la partie d'alignement (31) et la géométrie de tige (13).

8. Outil (3) selon l'une quelconque des revendications 1 à 7, **caractérisé en ce que** la douille (9) est réalisée en matière plastique ou **en ce que** des fentes sont prévues dans la douille (9), fentes qui sont fermées facultativement avec une masse de scellement ou des inserts en matière plastique.

9. Jeu d'instruments médicaux avec au moins un outil (3) selon l'une quelconque des revendications 1 à 8 et une poignée (1) avec un entraînement (15) et un mandrin d'outil, dans lequel la poignée (1) est prévue et réalisée afin de recevoir l'outil (3), **caractérisé en ce que** la poignée (1) présente une unité d'encliquetage et d'entraînement (19) qui fixe axialement l'outil (3) dans un état inséré dans la poignée (1) et par le biais de laquelle un couple d'entraînement est transmis à l'outil (3).

10. Jeu d'instruments médicaux selon la revendication 9, **caractérisé en ce que** l'unité d'encliquetage et d'entraînement (19) présente des tonneaux (35).

11. Jeu d'instruments médicaux selon la revendication 10, **caractérisé en ce que** le nombre de tonneaux (35) dans la poignée (1) correspond au nombre de creux (32) en forme de cavité.

12. Jeu d'instruments médicaux selon l'une quelconque des revendications 9 à 11, **caractérisé en ce que** la poignée (1) contient un élément descendant (21) qui présente une géométrie négative de la partie d'alignement et est prévu et réalisé afin de recevoir la partie d'alignement (31) de l'outil (3) dans un état encliqueté.

13. Jeu d'instruments médicaux selon la revendication 12, **caractérisé en ce que** l'élément descendant (21) oriente/positionne l'outil (3) lors d'une insertion de l'outil (3) dans la poignée (1) de manière prédéterminée de sorte que les tonneaux (35) viennent en prise dans les creux (32) en forme de cavité.

14. Jeu d'instruments médicaux selon l'une quelconque des revendications 9 à 13, **caractérisé en ce que** la poignée (1) contient une unité d'identification (25) qui est réalisée/montée de manière proximale par rapport à l'ouverture de poignée et de manière distale par rapport à l'unité d'encliquetage et d'entraînement (19) dans la tige (5) de la poignée (1).
